# EUROPÄISCHE PATENTSCHRIFT

(11) **EP 3 630 225 B1**
(45) Veröffentlichungstag und Bekanntmachung des Hinweises auf die Patenterteilung: **24.04.2024**
(21) Anmeldenummer: 18726983.2
(22) Anmeldetag: 23.05.2018
(51) Int. Cl.: A61M 5/142, A61M 39/24

(54) **INFUSIONSPUMPE MIT EINEM VERSCHIEDENE BETRIEBSZUSTÄNDE EINNEHMBAREN PUMPMODUL**
INFUSION PUMP COMPRISING A PUMP MODULE THAT CAN ASSUME DIFFERENT OPERATING STATES
POMPE À PERFUSION COMPORTANT UN MODULE DE POMPE POUVANT ADOPTER DIVERS ÉTATS DE FONCTIONNEMENT

(30) Priorität: 23.05.2017 DE 102017111299
(43) Veröffentlichungstag der Anmeldung: 08.04.2020
(73) Patentinhaber: B. Braun Melsungen AG, 34212 Melsungen (DE)
(72) Erfinder: FUCHS, Jürgen, 34308 Bad Emstal (DE); HARTUNG, Jürgen, 34298 Helsa (DE); STEGER, Jürgen, 34327 Körle (DE)
(74) Vertreter: Winter, Brandl - Partnerschaft mbB
(86) Internationale Anmeldenummer: PCT/EP2018/063510
(87) Internationale Veröffentlichungsnummer: WO 2018/215543

(56) Entgegenhaltungen:
- EP-A2- 0 110 276
- WO-A2-2010/048414
- GB-A- 2 126 666
- US-A1- 2011 150 680

## Beschreibung

Die vorliegende Erfindung betrifft ein Pumpmodul für eine medizintechnische Infusionspumpe zur Förderung von Fluid von einer Fluidquelle zu einem Patienten. Sie betrifft des Weiteren eine medizintechnische Infusionspumpe zur Förderung von Fluid von einer Fluidquelle zu einem Patienten, umfassend ein solches Pumpmodul.

### Allgemeiner technischer Hintergrund

In der Infusionstechnik sind allgemein Schlauchpumpen sowie Spritzenpumpen bekannt, um einem Patienten Flüssigkeit zuzuführen. Für Anwendungen, die im Hinblick auf die Dosiergenauigkeit hohe Anforderungen stellen, werden in der Regel Spritzenpumpen verwendet, da sich mit diesen eine definierte Fluidförderung gut erzielen lässt. Bei Anwendungen, die ein größeres Fördervolumen an Fluid verlangen, sind Schlauchpumpen gut geeignet, da sie in Folge ihrer Fördertechnik weitgehend kontinuierlich und ohne diskretes Fördervolumen fördern.

### Stand der Technik

Aus dem Stand der Technik sind solche Anwendungsfälle einer Infusionspumpe bekannt, in denen relativ hohe Fördervolumina gepaart werden müssen mit einer hohen Fördergenauigkeit, was zu großen Problemen führt. Spritzenpumpen weisen nämlich den Nachteil auf, dass das mit ihnen förderbare Fluidvolumen begrenzt ist durch das Volumen der verwendeten Spritze. Schlauchpumpen hingegen können zwar relativ große Fördervolumina schaffen, besitzen jedoch den Nachteil einer geringen Fördergenauigkeit. Insbesondere bei kleinen Förderraten ist ihre Fördergenauigkeit auf Grund der üblicherweise eingesetzten Peristaltik nicht gewährleistet. Außerdem ist der Antrieb einer Schlauchpumpe in der Regel relativ groß und schwer und verbraucht durch das Walken des Pumpenschlauchs durch den Peristaltikantrieb verhältnismäßig viel Energie.

Eine Verwendung von Spritzenpumpen mit entsprechend großem Volumen kann im Hinblick auf die vorstehende Problematik nur bedingt (wenn überhaupt) Abhilfe schaffen. Üblicherweise werden Spritzen mit einem Volumen von bis zu 50ml verwendet. Der verhältnismäßig große Kolbenquerschnitt einer Spritze mit großem Volumen bewirkt, dass hohe Kräfte zum Bewegen des Kolbens in der Spritze aufzubringen sind. Um derartige Spritzen oder gar solche mit einem größeren Volumen ausdrücken zu können, ist ein entsprechend leistungsfähiger und damit in der Regel großer, schwerer und kostenintensiver Antrieb erforderlich. Ein weiterer Nachteil ist, dass bekannte Spritzenpumpen eine einseitige Einspannung für die Spritze aufweisen, wodurch es zu einer Kräftezerlegung mit negativem Einfluss auf die Genauigkeit und Stabilität der Förderrate kommen kann. Bei Spritzenpumpen mit Einwegspritzen besteht außerdem der Nachteil, dass der darin verwendete Kolben nur einmal zum Füllen und Entleeren der Spritze verwendet werden kann.

Verschiedene Infusionspumpen sind in US201/150680 A1, EP0110276 A2, WO2010/048414 A2 und GB2126666 A beschrieben.

Insgesamt besteht daher im Stand der Technik der Nachteil, dass ein Anwender sich vor einer Therapie entscheiden muss, ob eine hohe Genauigkeit oder ein großes Fördervolumen benötigt wird. Somit muss der Anwender zwei unterschiedliche Infusionspumpen Typen vorhalten

### Kurzbeschreibung der Erfindung

Ausgehend von der vorstehend erläuterten Problematik liegt der Erfindung die Aufgabe zugrunde, die zuvor angeführten Nachteile zu beseitigen, insbesondere ein Infusionspumpsystem bereit zu stellen, das bekannte Infusions-Schlauch und - Spritzenpumpen ersetzen kann und universell insbesondere bei Anwendungen mit großer Volumenförderung und hoher Fördergenauigkeit einsetzbar ist. Vorzugsweise soll seine Baugröße im Vergleich zu bekannten Systemen gering sein. Es soll des Weiteren einfach und kostengünstig sein und vorzugsweise mittels eines Akkus betrieben werden können.

Diese Aufgabe wird nach der Erfindung gelöst durch eine Infusionspumpe mit den Merkmalen des Patentanspruchs 1. Vorteilhafte Ausgestaltungen der Erfindung sind Gegenstand der Unteransprüche.

Insbesondere wird die Aufgabe gelöst durch eine Infusionspumpe mit einem Pumpmodul umfassend eine Kolben-Zylinder-Einheit, die dafür angepasst ist, mittels eines Antriebsmechanismus der Infusionspumpe vorzugsweise bestehend aus einem elektrischen Antriebsmotor sowie ggf. einem Übersetzungsgetriebe zur Transformation der Rotationsbewegung des Antriebsmotors in eine Translationsbewegung/Hin-/HerBewegung des im Zylinder verschiebbar gelagerten Kolbens betätigt zu werden, der weiter vorzugsweise über eine Steuer-/Regeleinheit der Infusionspumpe gesteuert/geregelt ist, sowie mit einer Ventileinrichtung, welche vorzugsweise an die Steuer-/Regeleinheit angeschlossen ist und welche dafür angepasst ist, um ggf. in geregelter/gesteuerter Weise im Fall eines Pumpförderhubs eine Verbindung zwischen der Kolben-Zylinder-Einheit als Flüssigkeit-Zwischenspeicher und einem Versorgungsanschluss zu einem separaten Flüssigkeitsgroßvolumen zu unterbrechen sowie eine Verbindung zwischen der Kolben-Zylinder-Einheit und einem Patientenanschluss freizugeben und im Fall eines Pumpsaughubs die Verbindung zwischen der Kolben-Zylinder-Einheit und dem Versorgungsanschluss zu dem separaten Flüssigkeitsgroßvolumen freizugeben sowie die Verbindung zwischen der Kolben-Zylinder-Einheit und dem Patientenanschluss zu sperren.

Im einfachsten Fall hat die Ventileinrichtung passiv betätigbare Ventilelemente, d.h. die Ventileinrichtung hat bevorzugt zwei Rückschlagventile, welche in (separaten, als Einweg-Artikel ausgebildeten) Verbindungsleitungen zum Patientenanschluss und zum Flüssigkeitsgroßvolumen untergebracht sind, die in die Infusionspumpe einlegbar sind. Dies hat den Vorteil, dass die Ventileinrichtung besonders preisgünstig herstellbar ist und sich damit als Einweg-Artikel zusammen mit dem gesamten Flüssigkeitsleitungssystem entsorgt werden können.

Alternativ kann es vorgesehen sein, die Ventileinrichtung mit aktiv betätigbaren Ventilelementen, vorzugsweise zwei Schlauchquetschen/Pressen zu versehen, die auf flexibel deformierbare Abschnitte der beiden Verbindungsleitungen für deren dichtendes Abdrücken einwirken können und die bevorzugt in der Infusionspumpe (oder Pumpenmodul) untergebracht sind. Im Konkreten kann demnach das vorzugsweise als Einweg-Artikel ausgebildete Flüssigkeitsleitungssystem einen ersten, vorzugsweise elastisch verformbaren Schlauchabschnitt zur Fluidzufuhr (aus dem Flüssigkeitsgroßspeicher in den Zwischenspeicher) und einen zweiten, vorzugsweise elastisch verformbaren Schlauchabschnitt zur Fluidabfuhr (aus dem Zwischenspeicher in Richtung hin zum Patienten) haben, wobei die beiden Schlauchabschnitte für ein Einsetzen in die Infusionspumpe sowie für ein Anschließen an die Kolben-Zylinder-Einheit (Flüssigkeitszwischenspeicher) zum Ansaugen von Fluid aus dem ersten Schlauchabschnitt in den Flüssigkeitszwischenspeicher ausgebildet sind. Die Schlauchquetschen/Pressen können bevorzugt als Stempel/Stößel oder als Scherenklemmen oder dergleichen mechanische Klemmmittel ausgebildet sein, die beweglich in der Infusionspumpe gelagert sind (sind damit Bestandteil der Infusionspumpe) und von jeweils einem Antrieb betätigt werden können, der an die Steuer-/Regeleinheit angeschlossen ist. Diese Variante ermöglich ein kontrolliertes und damit sicheres Öffnen und Sperren der jeweiligen Flüssigkeits-/Fluidleitungen und auch ein preisgünstiger herstellbares Flüssigkeitsleitungssystem. Kennzeichnendes Merkmal hierbei ist, dass die beiden im Einlass und Auslass befindlichen Schlauchquetschen/Pressen derart mechanisch miteinander gekoppelt sind, dass mindestens eine Seite den Schlauch, sicher abdrückt. Damit beim Wechsel zwischen Einlass und Auslass keine undichte Position entsteht werden hierzu die Schlauchquetschen/Pressen mit z.B. Federn vorgespannt, so dass diese die ansonsten undichten Positionen überbrücken. Alternativ kann die Steuerzeit der Ventile auch über z.B. Kurvenscheiben oder durch getrennte Ansteuerungen mittels Motoren erfolgen.

Erfindungsgemäß kann die Kolben-Zylinder-Einheit, die insbesondere in Form einer Einwegspritze ausgebildet sein kann, mit dem ersten Schlauchabschnitt, als Zulaufleitung und dem zweiten Schlauchabschnitt, als patientenseitige Auslaufleitung versehen sein. Zumindest einer der oder beide Schlauchabschnitte können fest mit der Kolben-Zylinder-Einheit verbunden sein, beispielsweise einstückig, kraftschlüssig oder stoffschlüssig, zum Beispiel durch Anspritzen im Falle von Kunststoffteilen. Über den Antrieb, beispielsweise in Form eines Motors kann die Kolben-Zylinder-Einheit betätigt werden, das heißt entleert / ausgedrückt und gefüllt / aufgezogen werden.

Erfindungsgemäß kann die Kolben-Zylinder-Einheit mit einer zusätzlichen Dichtlippe versehen sein, die es erlaubt in Saugrichtung hohen Unterdrücken standzuhalten, ohne dass Luft die Dichtung zwischen Kolben und Zylinder überwinden kann. Anders gesagt, der Kolben hat jeweils eine Dichtung zum Abdichten gegen Überdruck und eine Dichtlippe zum Abdichten gegen Unterdruck.

Erfindungsgemäß kann der Kolben länger als der maximale Füllstand des Zylinders sein. Hierdurch wird vermieden, dass sich Umgebungsluft die sich naturgemäß außerhalb des Zylinders befindet mit dem im Zylinder befindlichen Medium vermischt. In der Kombination aus zwei Dichtlippen und Kolben / Zylinderlänge ergibt sich eine wirksame Barriere gegen eindringende Keime.

Über die Steuereinheit, die zum Beispiel in Form einer motorisch betriebenen Ventilsteuerung realisiert sein kann, kann der Schlauch im Auslauf, also der zweite Schlauchabschnitt, gesperrt, vorzugsweise abgedrückt werden, wenn die Kolben-Zylinder-Einheit / Spritze aufgezogen wird. Zudem kann der Schlauch im Zulauf, also der erste Schlauchabschnitt, gesperrt, vorzugsweise abgedrückt werden, wenn die Kolben-Zylinder-Einheit / Spritze ausgedrückt wird. Bezeichnend ist, dass die Kolben-Zylinder-Einheit / die Spritze wie eine Kolbenpumpe angesteuert werden kann. Insgesamt ist es durch die Erfindung möglich, bei Infusionsanwendungen im weitesten Sinne große Fluidmengen / Volumina mit der hohen Präzision einer Kolben-/Spritzenpumpe zu fördern. Die hohe Präzision ist dadurch bedingt, dass die Förderungsrate / Fördermenge aufgrund der mit der Kolben-Zylinder-Einheit durchgeführten Förderung sehr präzise ist. Änderungen des Fördervolumens, also des Volumens der Zylinderkammer, können in einem weiten Förderratenbereich genau eingestellt und angesteuert werden. Leckageströme können durch diese Bauart im Wesentlichen vollständig vermieden werden. Der Nachteil der bei Kolbenpumpen üblicherweise vorliegenden eingeschränkten Fördermenge wird im Sinne der Erfindung dadurch behoben, dass die Kolben-Zylinder-Einheit fortlaufend alternierend gefüllt und entleert wird, was durch die erfindungsgemäße Steuerung der Zu- und Abströmungen zur bzw. von der Kolben-Zylinder-Einheit ermöglicht wird. Eine Möglichkeit, eine weitgehend konstante Förderrate zu bewirken, besteht in der Kombination zweier erfindungsgemäßer Pumpen oder darin, die Pumpe mit zwei Kolben-Zylinder-Einheiten und diesen jeweils zugeordneten Steuereinheiten zu versehen, die phasenverschoben betrieben werden.

Vorteilhafte Ausführungsformen der Erfindung sind in den Unteransprüchen beansprucht und werden nachfolgend näher erläutert.

Nach einer Ausführungsform kann die Kolben-Zylinder-Einheit einen Zylinder und einen darin reziprok bewegbaren Kolben aufweisen. Der Kolben kann insbesondere an einer Kolbenstange, besonders bevorzugt an einem den beiden Schlauchabschnitten zugewandten Ende der Kolbenstange, angeordnet sein. Vorzugsweise ragt diese mit ihrem anderen Ende aus dem Zylinder heraus. Nach einer Ausführungsform weist die Kolben-Zylindereinheit ein Kuppelelement zum lösbaren Kuppeln mit einem entsprechenden Gegenelement eines Antriebs der Infusionspumpe auf. Dieses Kuppelelement kann direkt am Kolben angeordnet oder ausgebildet sein. Vorzugsweise ist es jedoch an dem den beiden Schlauchabschnitten abgewandten Ende der Kolbenstange angeordnet oder ausgebildet. Das Kuppelelement kann zum Beispiel in Form einer Steckkupplung ausgebildet sein und beim bestimmungsgemäßen Anordnen des Pumpmoduls in der Infusionspumpe automatisch oder zwangsläufig mit dem Antrieb gekoppelt werden. Vorzugsweise weist die Kupplung Raststrukturen auf, die beide Kuppelelemente aneinander halten und insbesondere ein für einen Anwender hörbares und/oder fühlbares Verrasten der beiden Kuppelelemente ermöglichen.

Bei der Kolben-Zylinder-Einheit handelt es sich vorzugsweise um eine leichtgängige Einheit, um Betätigungskräfte gering zu halten und eine hohe Fördergenauigkeit zu erzielen. Dies kann beispielsweise bewirkt werden, indem der Kolben in Achsrichtung beidseitig mit Dichtungen versehen und derart gegenüber dem Zylinder abgedichtet ist. Insbesondere kann die Kolben-Zylinder-Einheit in Form einer Spritze mit einem leichtgängigen Kolben ausgebildet sein, der Dichtkonturen in Zug- und Druckrichtung aufweist. Hierdurch ist die Verwendung des Kolbens unter Beibehaltung der Dichtwirkung in beide Bewegungsrichtungen möglich.

Erfindungsgemäß ist der Kolben länger als der maximale Füllstand des Zylinders hierdurch wird vermieden, dass sich Umgebungsluft die sich naturgemäß außerhalb des Zylinders befindet mit dem im Zylinder befindlichen Medium vermischt. In der Kombination aus zwei Dichtlippen und Kolben / Zylinderlänge ergibt sich eine wirksame Barriere gegen eindringende Keime.

Eine bevorzugte Ausführungsform des Pumpmoduls sieht vor, dass der erste Schlauchabschnitt am der Kolben-Zylinder-Einheit gegenüberliegenden Ende mit einem Luer-Lock-Kupplungsstück versehen ist. Dieses kann insbesondere als Luer-Lock-Innenkegel ausgebildet sein. Alternativ oder zusätzlich kann der zweite Schlauchabschnitt am der Kolben-Zylinder-Einheit gegenüberliegenden Ende mit einem Luer-Lock-Kupplungsstück versehen sein. Dieses kann insbesondere als Luer-Lock-Außenkegel ausgebildet sein. Dies ermöglicht eine besonders einfache zu betätigende Möglichkeit zur Verbindung des Pumpmoduls bzw. dem darin aufgenommenen Flüssigkeitsleitungssystem mit einer extrakorporalen Leitung oder Leitungssystem. Die Erfindung lässt sich damit ohne weiteres und in bekannter Weise zusammen mit bestehenden und weitverbreiteten medizintechnischen Einrichtungen verwenden. Alternativ zu einem Luer-Lock-Anschluss kann eine am Patienten angeschlossene Tropfkammer vorgesehen sein oder der zweite Schlauchabschnitt ist als Sackleitung mit einem verschlossen Ende ausgebildet und kann mit einem Einsteckdorn angeschlossen werden. Zusätzlich oder alternativ bietet sich auch das Anordnen eines Spikes / Einstechdorns an, um die Flexibilität im Anwendungsgebiet bzw. die Kompaktheit der Pumpe zu erhöhen.

Eine bevorzugte Ausführungsform der Infusionspumpe nach der Erfindung ist dadurch gekennzeichnet, dass die Steuereinheit und der Antrieb an/in einem Gehäuseteil der Infusionspumpe angeordnet sind. Das Pumpmodul kann insbesondere anwenderseitig austauschbar an dem Gehäuseteil anzuordnen sein. Dabei ist es vorzugsweise ohne Nutzung besonderer Werkzeuge oder Vorrichtungen in der Pumpe anzuordnen und/oder aus dieser zu entfernen. Das erfindungsgemäße Pumpmodul kann insbesondere als Einmalartikel ausgebildet und für einzige Verwendung in der Pumpe bestimmt sein. Dies ist im Hinblick auf Sterilbedingungen besonders vorteilhaft und anwenderfreundlich. Es kann zum Beispiel in Form einer Einmalspritze realisiert sein, mit deren Fördervolumenöffnung die beiden Schlauchabschnitte strömungstechnisch verbunden sind. Dies kann derart realisiert sein, dass die beiden Schlauchabschnitt eine fortlaufende Fluidleitung ausbilden, die in einem Bereich zwischen den Deformationsstellen mit einer Abzweigung zum Anschließen der Kolben-Zylinder-Einheit, insbesondere einer Einmalspritze, versehen ist. Alternativ kann die Kolben-Zylinder-Einheit / die Einmalspritze zwei Strömungsöffnungen aufweisen, einen Ausgang und einen Eingang, die jeweils strömungstechnisch mit dem entsprechenden Schlauchabschnitt verbunden werden.

Die Erfindung ist dadurch gekennzeichnet, dass die Ventilsteuerung derart mit dem Antrieb für die Kolben-Zylinder-Einheit gekoppelt ist, dass sie ein im Wesentlichen fluiddichtes Zusammenquetschen des ersten Schlauchabschnitts (und ein Freigeben des Strömungsquerschnitts des zweiten Schlauchabschnitts) bewirkt, wenn Fluid aus der Kolben-Zylinder-Einheit ausgetrieben wird. Des Weiteren bewirkt sie ein im Wesentlichen fluiddichtes Zusammenquetschen des zweiten Schlauchabschnitts (und ein Freigeben des Strömungsquerschnitts des ersten Schlauchabschnitts) bewirkt, wenn Fluid in die Kolben-Zylinder-Einheit eingesaugt wird. Auf diese Weise kann besonders einfach eine fortlaufende Förderung bewirkt werden, wobei jeweils ein Einlasszyklus / Einsaugzyklus in die Zylinderkammer und ein Auslasszyklus / Ausdrückzyklus aus der Zylinderkammer alternierend ablaufen. Die beiden im Einlass und Auslass befindlichen Schlauchquetschen/Pressen sind derart mechanisch mit einander gekoppelt, dass mindestens eine Seite den Schlauch sicher abdrückt. Dies damit beim Wechsel zwischen Einlass und Auslass keine undichte Position entsteht. Hierzu sind die Schlauchquetschen/Pressen mit z.B. Federn vorgespannt, so dass diese die ansonsten undichten Positionen überbrücken. Alternativ kann die Steuerzeit der Ventile auch über z.B. Kurvenscheiben oder durch getrennte Ansteuerungen mittels Motoren erfolgen

Es ist von besonderem Vorteil, wenn der Antrieb der Kolben-Zylinder-Einheit ein Linearantrieb/-motor ist, der in der Bewegungsachse des Kolbens angeordnet ist. Besonders bevorzugt ist, wenn der Antrieb derart angeordnet und ausgebildet ist, dass vom Antrieb auf den Kolben wirkende Kräfte zentral und in Achsrichtung eingeleitet werden. Dabei ist von besonderem Vorteil, dass auf den Kolben wirkende Kräfte minimiert werden können (im Vergleich zu einer dezentralen Krafteinleitung) und derart nur eine geringe Energie zur Betätigung der Kolben-Zylinder-Einheit erforderlich ist. In der Folge kann der Antrieb und damit die Pumpe klein ausgebildet sein, was zu Einsparungen hinsichtlich Gewicht, Kosten und Bauraum führt. Außerdem kann durch die zentrale Einleitung von Betätigungskräften in den Kolben eine hohe Präzision erzielt werden. Die Verwendung einer kleinen Spritze als Kolben-Zylinder-Einheit ermöglicht eine Anordnung eines Linearantriebs unmittelbar in der Achse des Spritzenkolbens, wodurch erreicht wird, dass keine die Genauigkeit beeinflussenden Querkräfte auf den Spritzenkolben wirken. Des Weiteren erzeugt ein kleiner Querschnitt der Spritze nur geringe Kräfte, was eine Verwendung eines einfachen kostengünstigen Antriebs, zum Beispiel eines Linear-Schrittmotors, ermöglicht. Die Baugröße der Pumpe kann dadurch deutlich geringer ausfallen als bei bekannten Infusionspumpen.

Die erfindungsgemäße Pumpe weist vorzugsweise einen zweiten Antrieb, insbesondere Linearantrieb/-motor für die Steuereinheit auf. Die beiden Antriebe für die Kolben-Zylinder-Einheit und die Steuereinheit sind steuerungstechnisch miteinander gekoppelt, derart, dass die vorstehend beschriebene Steuerungsfunktion durch Abquetschen der beiden Schlauchabschnitte erfolgt.

Nach einer weiteren bevorzugten Ausführungsform kann die Pumpe eine Aufnahme für das Pumpmodul aufweisen. Diese kann beispielsweise in Form einer Ausnehmung in einem Pumpengehäuse ausgebildet sein und insbesondere mittels eines Verschlusses (zum Beispiel in Form einer schwenkbar an der Pumpe, insbesondere an deren Gehäuse, angeordneten Verschlussklappe) zu verschließen sein. Der Verschluss kann insbesondere in einer die Aufnahme verschließenden Stellung über eine Verriegelungseinheit mit dem Gehäuseteil verriegelbar sein. Der Verschluss, die Aufnahme und das Pumpmodul können derart aufeinander abgestimmt sein, dass ein Schließen (und ggf. Verriegeln) des Verschlusses nur möglich ist, wenn das Pumpmodul in bestimmungsgemäßer Weise und korrekt angeordnet und angeschlossen ist. Derart kann einem Nutzer ein Feedback im Hinblick auf eine fehlerfreie Einrichtung und Nutzung der Pumpe gegeben werden, was in vorteilhafter Weise die Patientensicherheit erhöht. Außerdem kann durch ein Schließen der Verschlussklappe ein automatisches Kuppeln der Kolben-Zylinder-Einheit mit dem Antrieb bewirkt werden, was eine besonders einfache und sichere Bedienung darstellt. Ein Gehäuse der Pumpe kann insbesondere aus einem Gehäuseunterteil, an dem die gesamte Mechanik und Elektronik angeordnet und gehalten sein kann, sowie aus einem Gehäuseoberteil, das insbesondere ein Display und diversen Schaltelemente aufweisen kann, bestehen. Im Inneren des Gehäuses kann sich ein Verschlussmechanismus für die Verschlussklappe befinden.

Die Infusionspumpe ist dadurch gekennzeichnet, dass die Steuereinheit eine motorisch angetriebene Kipphebeleinheit hat. Diese kann derart ausgebildet sein und wirken, dass sie in einer ersten Kippstellung den ersten Schlauchabschnitt zusammenquetscht und den zweiten Schlauchabschnitt freigibt und in einer zweiten Kippstellung den zweiten Schlauchabschnitt zusammenquetscht und den ersten Schlauchabschnitt freigibt.

Die Steuereinheit kann insbesondere einen schwenkbar an der Kipphebeleinheit angeordneten Druckstempel aufweisen. Sie kann vor allem einen mit dem ersten Schlauchabschnitt zusammenwirkenden Einlassstempel und/oder einen mit dem zweiten Schlauchabschnitt zusammenwirkenden Auslassstempel umfassen. Zumindest ein Druckstempel kann mittels einer Vorspanneinheit, insbesondere mittels einer Druckfeder, in eine den jeweiligen Schlauchabschnitt freigebenden, insbesondere nicht kontaktierende, Position vorgespannt sein. Auf diese Weise kann bewirkt werden, dass sich die Druckstempel nicht im Bereich des entsprechenden Schlauchabschnitts befinden, wenn die Pumpe nicht in Betrieb ist. Dies ermöglicht ein besonders anwenderfreundliches Wechseln, Einsetzen und Entfernen des erfindungsgemäßen Pumpmoduls.

Da der Antrieb / die Antriebe der Pumpe klein ausgelegt sein können, ist es in vorteilhafter Weise möglich, dass die Pumpe eine Energiespeichereinheit, insbesondere einen Akku, umfasst und ohne direkten Netzanschluss betrieben werden kann. Die Energiespeichereinheit kann zum Beispiel ein üblicher Lithium-Akku sein, der über eine USB-Schnittstelle geladen werden kann. Diese kann außerdem genutzt werden, um Daten in eine Pumpensteuerung ein- und/oder auszulesen.

Bevorzugt ist die beidseitige Dichtung des Kolbens der Kolben-Zylinder-Einheit mittels einer proximalen und einer distalen Dichtung realisiert. So ist eine Unterdruckbewegung und eine Überdruckbewegung des Kolbens abgedichtet, um eine Sterilität eines Fluidraums / Dosierraums, das heißt des Raums, in dem die sich die zu fördernde Flüssigkeit im Zylinder aufhält, zu gewährleisten. So erfüllt die Pumpe die hohen Anforderungen hinsichtlich der Sauberkeit in medizinischem Umfeld.

Die proximale und die distale Dichtung verlaufen zueinander vorteilhafterweise planparallel. Da der Kolben im Wesentlichen einen runden Querschnitt aufweist, resultieren hieraus etwa ringförmige Dichtungen. Die Distanz zwischen der der proximalen und der distalen Dichtung ist insbesondere größer als der Förderhub der Kolben-Zylinder-Einheit ausgestaltet. Hieraus resultiert, dass der Dosierbereich ausschließlich mit dem Kolben und dessen steriler Dichtlippe in Kontakt ist, wodurch eine Verschmutzung / Verunreinigung des Fluidraums / Dosierraums aufgrund zu hoher Hub-Bewegungen konstruktiv unterbunden ist.

Vorteilhafterweise weist das Pumpmodul eine Schiebeklemme auf, die derart einstellbar, das heißt verschiebbar ist, dass sie einen Fluidfluss in dem ersten und/oder dem zweiten Schlauchabschnitt wahlweise freigibt oder sperrt. So ist die Schiebeklemme derart einstellbar, dass sich nach der Inbetriebnahme des Pumpmoduls keine Luftblasen bilden. Die Schiebklemme ist vorzugsweise auf einen Griffabschnitt des Pumpmoduls aufclipsbar, sodass diese leicht aus einem anderen Material herstellbar ist als das restliche Pumpmodul.

Eine vorteilhafte Ausführungsform der Erfindung zeichnet sich dadurch aus, dass an einem der Schiebeklemme abgewandten Ende des Pumpmoduls bzw. des Griffabschnitts eine Hülse / ein Ring angeordnet ist. Diese Hülse ist dazu in der Lage, den ersten und/oder den zweiten Schlauchabschnitt zu fixieren und somit eine sichere Kopplung einer Infusionsleitung mit dem Pumpmodul zu ermöglichen. Sie dient demnach als Haltering, durch den die Infusionsleitung einfädelbar ist.

Zusammenfassend kann man sagen, dass die Erfindung ein Infusionspumpsystem mit der Genauigkeit einer Spritzenpumpe und dem Fördervolumen oder Volumenvorrat einer Schlauchpumpe bei deutlich geringerem Energiebedarf, reduzierter Baugröße und geringen Herstellungskosten ermöglicht. Durch die Erfindung können insbesondere die folgenden Vorteile erzielt werden:
- "One fits all": eine große Bandbreite an Infusionstherapien (nahezu alle bekannten) können mit nur einer einzigen Pumpe ausgeführt werden.
- Kostengünstige und kleine Infusionspumpe
- Die Pumpe kann an beliebigen Orten (überall) eingesetzt werden, insbesondere ohne Netzanschluss (mobil lange Akkulaufzeit), was sie besonders für Außeneinsätze oder für Einsätze in weniger entwickelten Gebieten qualifiziert.
- Die Pumpe ermöglicht eine genauere Dosierung der Infusion als übliche Infusionspumpen
- Feste Bindung des insbesondere als Einmalartikel ausgebildeten Pumpmoduls an die Pumpe
- Das Pumpmodul ist mit verschiedenen Arten von Infusionsleitungen koppelbar.

Der Antrieb für die Kolben-Zylinder-Einheit kann mit einer nicht dargestellten Drehüberwachung ausgerüstet sein. Hierdurch ist möglich, Störungen beim Zuführen des Fluids zu einem Patienten festzustellen, zum Beispiel einen Verschluss im Auslauf. Derartige Störungen können über Abweichungen des Antriebsverhaltens von üblichen Werten erkannt werden, zum Beispiel über ein Blockieren oder Verlangsamen des Motors. Mittels einer solchen Drehüberwachung kann auf eine Verwendung von ansonsten erforderlichen teuren Drucksensoren verzichtet werden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine perspektivische Darstellung einer Infusionspumpe nach der Erfindung mit eingesetztem Pumpmodul;
Fig. 2 eine perspektivische Darstellung einer erfindungsgemäßen Infusionspumpe mit eingesetztem Pumpmodul bei teilweise freigeschnittenem Pumpengehäuse;
Fig. 3 eine perspektivische Darstellung eines erfindungsgemäßen Pumpmoduls mit angeschlossenem Schlauch;
Fig. 4 eine perspektivische Darstellung einer Infusionspumpe sowie eines Pumpmoduls nach der Erfindung beim Einsetzen des Pumpmoduls in die Pumpe;
Fig. 5 eine detaillierte Ansicht des Pumpmoduls mit integrierter Schiebeklemme;
Fig. 6 das Pumpmodul mit daran angeschlossener Tropfkammer;
Fig. 7 das Pumpmodul mit daran angeschlossenem Spike;
Fig. 8 das Pumpmodul mit daran angeschlossenem Luer-Lock-Innenkegel; und
Fig. 9 das Pumpmodul mit dem Spike aus Fig. 7 in die erfindungsgemäße Infusionspumpe eingesetzt.

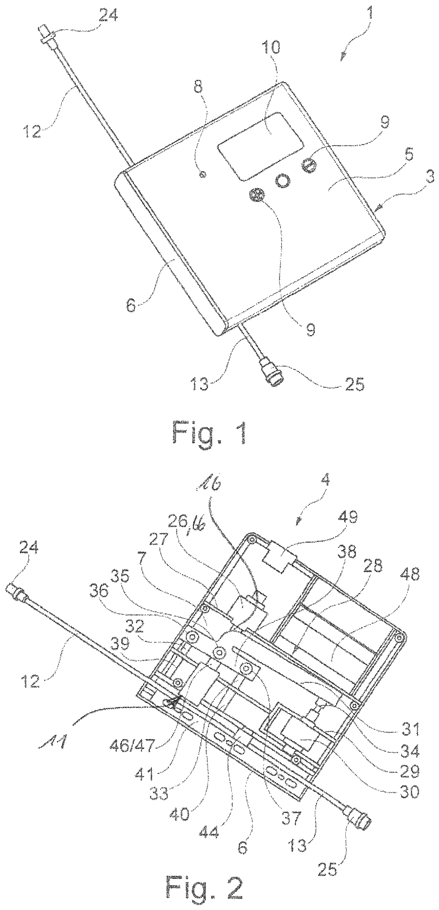

Figur 1 zeigt ein Ausführungsbeispiel einer Infusionspumpe 1 nach der Erfindung mit eingesetztem Pumpmodul 2. Die Pumpe 1 weist ein Gehäuse 3 auf, das im Wesentlichen bevorzugt aus einem Gehäuseunterteil 4 mit einem Gehäusedeckel 5 und einer Verschlussklappe 6 besteht. Das Gehäuseunterteil 4 und der Gehäusedeckel 5 sind miteinander verbindbar, zum Beispiel über in den Figuren nicht dargestellte Rastverbindung oder Verschraubungen, und bilden im zusammengefügten Zustand das Gehäuse 3 aus. Das Gehäuseunterteil 4 weist einen Gehäuseboden 7 auf. Im oder am Gehäusedeckel 5 sind im nachfolgenden beschriebene Einheiten/Elemente der Pumpe 1 angeordnet:
- Anzeigeelemente 8,
- Bedienungselemente 9 und ein
- Anzeigedisplay 10.

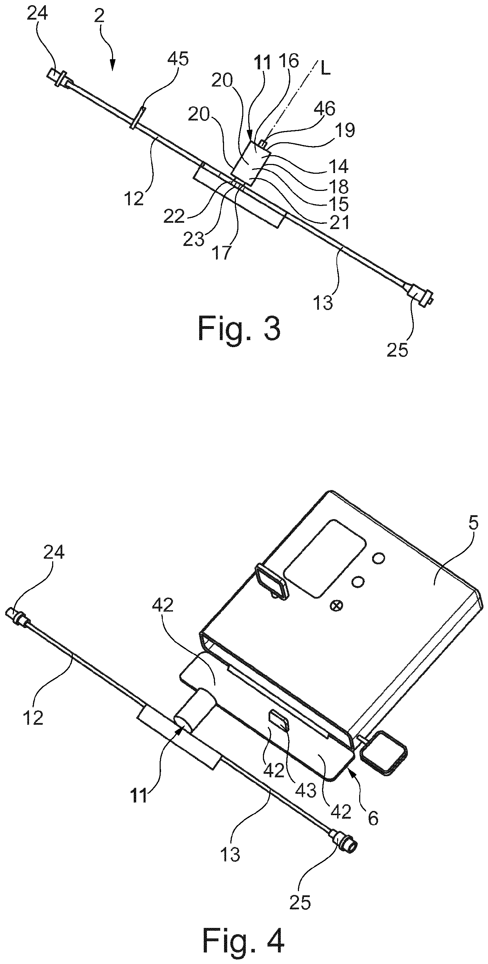

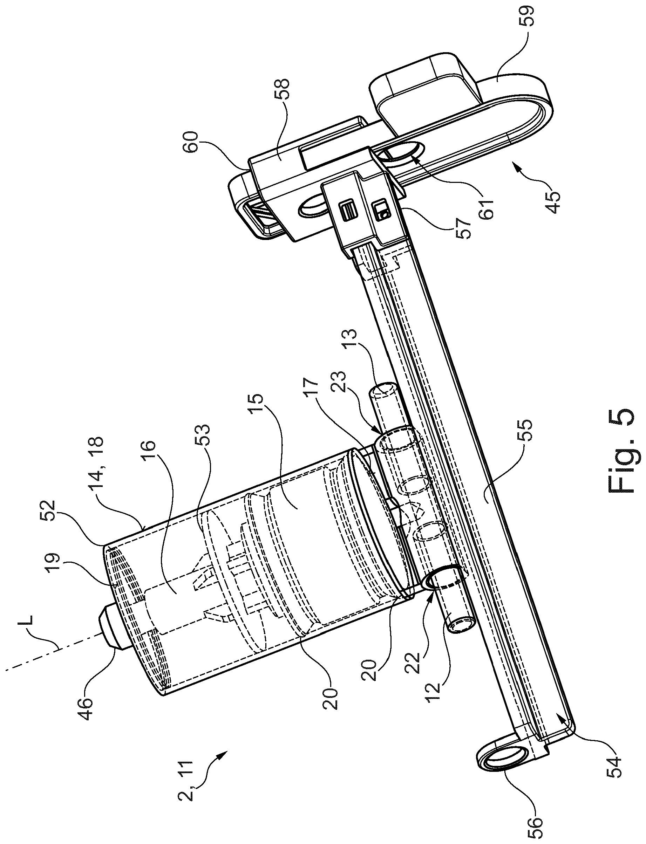

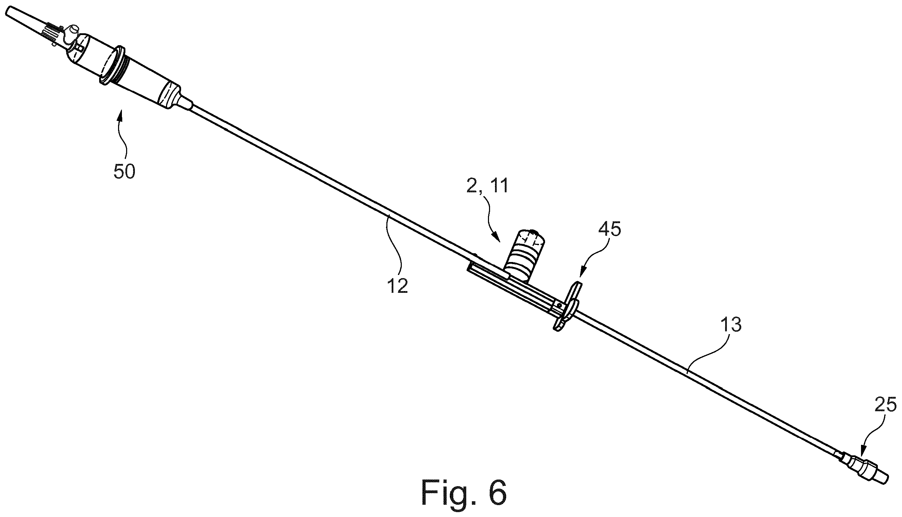

Das Pumpmodul 2 ist einzeln schematisch in Figur 3 gezeigt. Eine detailliertere Ausführungsform des Pumpmoduls 2 findet sich in Figur 5 wieder. Auch in den Figuren 6 bis 8 ist das Pumpmodul 2 dargestellt. Es weist eine Kolben-Zylinder-Einheit 11, einen ersten elastisch verformbaren Schlauchabschnitt 12 und einen zweiten elastisch verformbaren Schlauchabschnitt 13 auf. Das Pumpmodul 2 ist als Einmalartikel (Single Use Artikel, Wegwerfartikel) ausgebildet. Die Schlauchabschnitte 12, 13 können insbesondere als PVC-Schlauch ausgebildet sein.

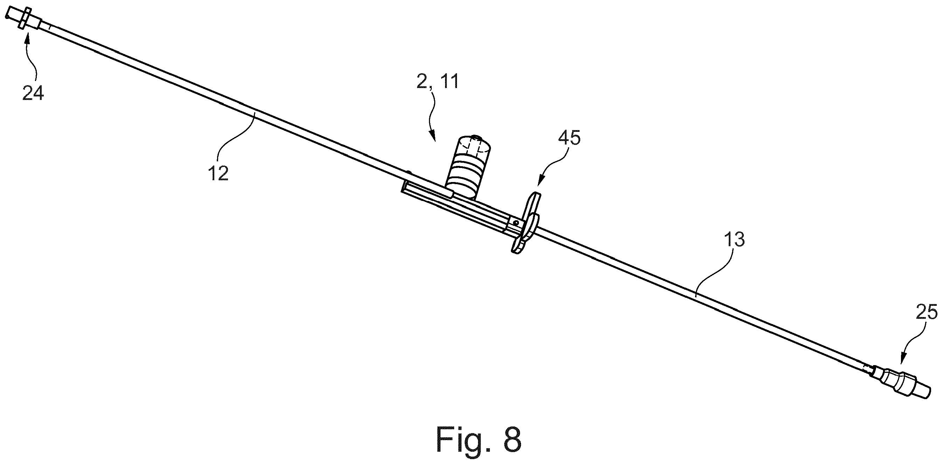

Die Kolben-Zylinder-Einheit 11 hat einen Zylinder 14, der insbesondere aus Kunststoff bestehen und/oder als Spritzgussteil hergestellt sein kann, und einen darin reziprok bewegbaren Kolben 15, der ebenfalls insbesondere aus Kunststoff bestehen und/oder als Spritzgussteil hergestellt sein kann. Der Kolben 15 ist auf/an einer Kolbenstange 16 angeordnet und zu dieser zumindest in axialer Richtung L lagefixiert. Der Zylinder 14 weist eine Bodenwand 17, eine Umfangswand 18 und eine Deckelwand 19 auf. Die Kolbenstange 16 ragt durch eine in der Deckelwand 19 ausgebildete Öffnung in axialer Richtung L aus dem Zylinder 14 heraus. Der Kolben 15 ist bevorzugt mittels beiderseits daran angeordneten Dichtungen 20 gegenüber der Umfangswand 18 abgedichtet, so dass die Kolben-Zylinder-Einheit 11 einen Fluidraum / Dosierraum 21 aufweist. In der Bodenwand 17 sind eine Einlaufbohrung 22 und eine Auslaufbohrung 23 ausgebildet. Der Fluidraum 21 ist über die Einlaufbohrung 22 strömungstechnisch mit dem ersten Schlauchabschnitt 12 und über die Auslaufbohrung 23 strömungstechnisch mit dem zweiten Schlauchabschnitt 13 verbunden. Die Schlauchabschnitte 12, 13 können insbesondere an den Zylinder 14 angeformt sein. Sie können insbesondere wie übliche Infusionsschläuche ausgebildet sein. Der erste Schlauchabschnitt 12 ist in der Ausführungsform aus Figur 3 an seinem von der Kolben-Zylinder-Einheit 11 abgewandten Ende mit einem Einlass-Luer-Lock-Anschluss 24 versehen, der vorliegend, wie auch in Fig. 8 als Luer-Lock-Innenkegel ausgebildet ist. Der zweite Schlauchabschnitt 13 ist in der Ausführungsform aus Figur 3 sowie in den Ausführungsformen in den Figuren 6 bis 9 an seinem von der Kolben-Zylinder-Einheit 11 abgewandten Ende mit einem Auslass-Luer-Lock-Anschluss 25 versehen, der vorliegend als Luer-Lock-Außenkegel ausgebildet ist. Damit ist das Pumpmodul 2 einfach an übliche Infusionseinheiten anzuschließen.

Figur 2 zeigt, dass die Pumpe 1 einen (pumpeneigenen) Antrieb 26, 66 für die Kolben-Zylinder-Einheit 11 aufweist, hier in Form eines Linearmotors 26 mit einem Übersetzungsgetriebe 66. Der Motor 26 ist kongruent zur Längsachse L der Kolbenstange 16 (der Kolben-Zylinder-Einheit 11) angeordnet, so dass Kräfte zum reziproken Antreiben des Kolbens 15 zentral und ohne Verwindungen / Querkräfte übertragen werden können. Der Antrieb 26 ist über eine Montage in einer Zwischenwand 27 lagefixiert am Gehäuseunterteil 4 angeordnet. Die Kolbenstange 16 ist mittels eines daran angeordneten oder ausgebildeten Kuppelelements 46 und eines zum Eingriff damit ausgebildeten Gegenelements 47 mit dem Antrieb 26, 66 verbindbar. Das Kuppelelement 47 dient demnach als Aufnahme für den Antrieb / Linearmotor 26.

Figur 2 zeigt des Weiteren, dass die Pumpe 1 eine (pumpeneigene) Steuereinheit 28 zumindest zum steuernden Zusammenquetschen der Schlauchabschnitte 12, 13 aufweist. Die Steuereinheit 28 ist wirktechnisch mit dem ersten Schlauchabschnitt 12 und dem zweiten Schlauchabschnitt 13 verbindbar und über eine weitere Zwischenwand 29 am Gehäuseunterteil 4 angeordnet. Sie hat eine mittels eines (angesteuerten/geregelten) Antriebs 30, hier eines Linearmotors 30, motorisch angetriebene Kipphebeleinheit mit einem Kipphebel 31 und zwei schwenkbar daran angeordneten Druckstempeln 32, 33 (Ventileinrichtungen). Der Kipphebel 31 ist über einen Mitnehmer 34 mit dem Linearmotor 30 verbunden und über ein Kipplager 35 um dessen in Figur 2 orthogonal zur Zeichnungsebene und in der Figur nicht gezeigte Kippachse schwenkbar gelagert. Die Druckstempel 32, 33 sind schwenkbar mittels Schwenklagern 36, 37 an einem Querarm 38 des Kipphebels 31 angeordnet und am Gehäuseunterteil 4 linearpositionierbar geführt. Sie sind mittels Druckfedern 39, 40 vorgespannt, derart, dass die Spannung der Druckfedern 39, 40 sie von den beiden Schlauchabschnitten 12, 13 fort drängt.

Figur 2 zeigt, dass die Druckstempel 32, 33 orthogonal zu den Schlauchabschnitten 12, 13 angeordnet sind. Sie sind des Weiteren in einer Richtung orthogonal zu den Schlauchabschnitten 12, 13 linearpositionierbar. Die Druckstempel 32, 33 sind derart ausgebildet und angeordnet und dazu vorgesehen, in einer ersten Kippstellung, die in Figur 2 gezeigt ist, den ersten Schlauchabschnitt 12 zusammen zu quetschen und den zweiten Schlauchabschnitt 13 freizugeben. In einer zweiten Kippstellung, die in keiner der Figuren gezeigt ist, ist der einlaufschlauchseitige Druckstempel 32 aus der in Figur 2 gezeigten Lage vom ersten Schlauchabschnitt 12 fort positioniert, also quasi in Richtung des Motors 26 verlagert, während der auslaufschlauchabschnittseitige Druckstempel 33 in Richtung des zweiten Schlauchabschnitts verlagert ist und diesen zusammenquetscht. In dieser Lage ist der erste Schlauchabschnitt 12 nicht mehr durch den Druckstempel 32 zusammengequetscht und sein Durchflussquerschnitt ist freigegeben. Der Linearmotor 30 ist in der in Figur 2 gezeigten Situation, in der der erste Schlauchabschnitt zusammengequetscht wird, ausgefahren. In der vorstehend beschriebenen und nicht dargestellten Funktionsstellung, in der der zweite Schlauchabschnitt 13 zusammengequetscht ist, ist er eingefahren. Durch reziprokes Ein-und Ausfahren des Motors 30 wird der Kipphebel 31 zu einer Kippbewegung um sein Lager 35 angeregt, durch die die beschriebene Verlagerung der Druckstempel 32, 33 bewirkt wird.

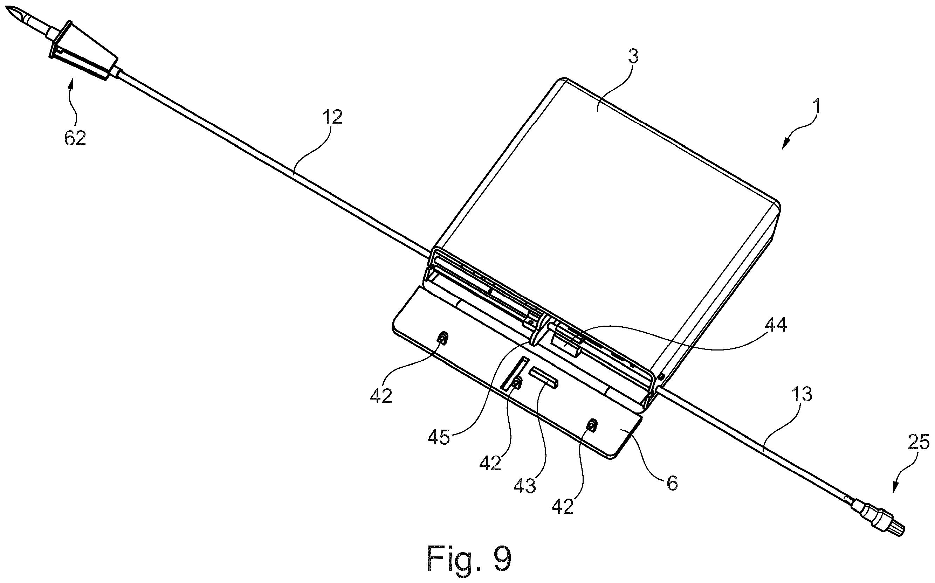

Aus den Figuren 1, 2, 4 und 9 ergibt sich, dass die Verschlussklappe 6 zwischen einer geöffneten Stellung (Figuren 4 und 9) und einer geschlossenen Stellung (Figuren 1 und 2) schwenkbar am Gehäuseunterteil 4 angeordnet ist. Sie ist mittels eines Verriegelungsmechanismus 41 am Gehäuse 3 verriegelbar und weist zu diesem Zweck mit dem Mechanismus 41 zusammenwirkende Riegelstifte 42 auf. Sie ist des Weiteren mit einem Positionierungszapfen 43 versehen, der mit dem zweiten Schlauchabschnitt 13 zusammenwirkt und sicherstellt, dass dieser bei geschlossener Verschlussklappe 6 bestimmungsgemäß an einem Luftsensor 44 platziert ist. Die Antriebe 26, 28 der Pumpe werden über eine Akku 48, der über einen USB-Anschluss 49 aufzuladen ist, energetisch versorgt.

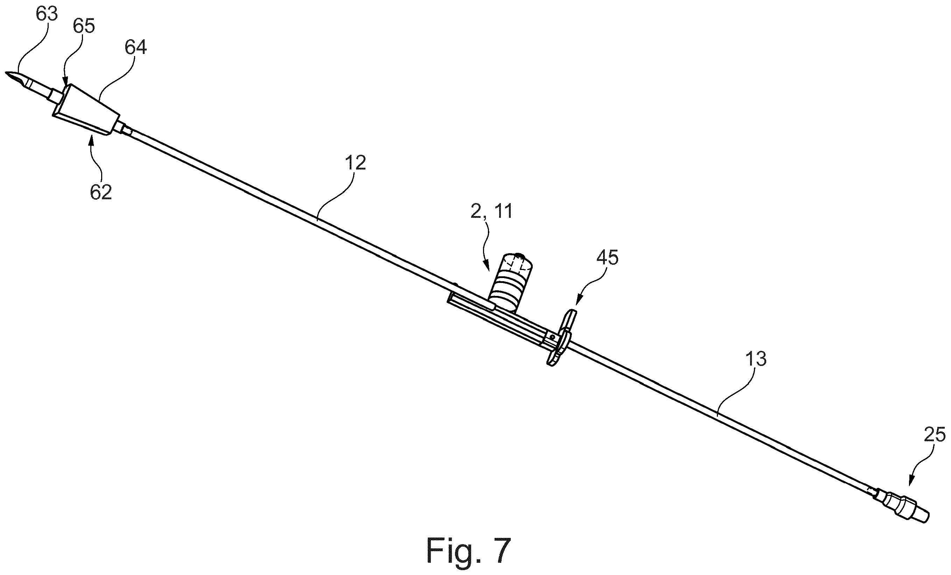

Zum Verwenden der Pumpe 1 öffnet ein Anwender zuerst die Verschlussklappe 6 der Pumpe 1, zum Beispiel indem er einen Spezialschlüssel seitlich in eine dafür vorgesehene Bohrung einführt und die Klappe 6 damit entriegelt. An dem Luer-Lock-Anschluss 24 des Pumpmoduls 2 aus den Figuren 1 bis 4 sowie 8 kann man beispielsweise ein Infusionsgerät anschließen Weiterhin ist das Pumpenmodul 2 mit einer Tropfkammer 50 ausgestattet (Figur 6) oder mit einem Spike 51 ausgestattet (Figuren 7 und 9), die ebenfalls Bestandteile von als Einwegartikel ausgestalteten Infusionsleitungen sein können. Nachdem die Infusionsleitung einschließlich der beiden Schlauchabschnitte 12, 13 bis zum Auslass-Luer-Lock-Anschluss 25 / der Tropfkammer 50 / dem Spike 51 luftblasenfrei befüllt ist, wird der erste Schlauchabschnitt 12 (Figur 3) bzw. der zweite Schlauchabschnitt 13 (Figuren 5 bis 9) mittels einer Schiebeklemme 45 fluiddicht verschlossen. Danach schiebt der Anwender das Pumpmodul 2 in die dazu vorgesehene Aufnahme der Pumpe 1, bis das Kuppelelement 46 der Kolbenstange 16 mit dem Gegenelement 47 des Antriebs 26 hör- und/oder fühlbar einrastet und die Kolben-Zylinder-Einheit 11 mit dem Antrieb 26 verbunden ist. Nachfolgend wird die Verschlussklappe 6 verschlossen. Der im Gehäuseoberteil 5 befindliche Verschlussmechanismus 41 sorgt für eine sichere Arretierung der Klappe 6. Gleichzeitig wird der zweite Schlauchabschnitt 13 bestimmungsgemäß zum Luftsensor platziert, zum Beispiel an diesen gedrückt. Ein Erkennen und entsprechendes Kommunizieren von eventuell vorhandenen oder eingebrachten Luftblasen bei einer Infusion ist damit sichergestellt.

Jetzt gibt der Anwender die gewünschte Förderrate über die Anzeigeelemente 8 und die Bedienungselemente 9 ein. Deren Darstellung erfolgt über das Display 10, das nach einer Ausführungsform als Touch-Screen ausgebildet sein kann, so dass eine Eingabe auch über das Display 10 erfolgen kann (eine Bedienung über eine App im Handy oder Tablet ist außerdem möglich und im Rahmen der Erfindung). Unmittelbar nach Freigabe startet die Pumpe 1. Der Linearmotor 30 fährt aus der in Figur 2 gezeigten Stellung zurück und der Auslaufstempel 33 klemmt den zweiten Schlauchabschnitt 13 fluiddicht ab. Gleichzeitig öffnet der Einlaufstempel 32 den ersten Schlauchschnitt12. Danach fährt der Linearmotor 26 für den Kolben 15 aus der in Figur 2 gezeigten Stellung nach hinten und saugt Infusionslösung durch den ersten Schlauchabschnitt 12 in die Fluidkammer 21 der Kolben-Zylinder-Einheit 11 ein. Sobald die eingestellte Dosierung erreicht ist, fährt der Linearmotor 30 zurück nach vorn in die in Figur 2 gezeigte Stellung und der Einlaufstempel 32 verschließt den ersten Schlauchabschnitt 12 fluiddicht. Gleichzeitig öffnet der Auslaufstempel 33 den zweiten Schlauchabschnitt 13. Danach fährt der Linearmotor 26 bis zum Anschlag zurück in die in Figur 2 gezeigte Stellung nach vorn und drückt die in der Fluidkammer 21 befindliche Infusionsflüssigkeit in den zweiten Schlauchabschnitt13. Dieser Vorgang wird bis zum Erreichen der vorgegebenen Dosierung fortgeführt. Nach Beendigung der Infusion wird die Verschlussklappe 6 wieder mit dem Spezialschlüssel geöffnet. Außerdem kann mittels des Spezialschlüssels eine Entriegelung der Kupplung zwischen Antrieb 26 und Kolbenstange 16 erfolgen. Nun kann das Pumpmodul 2 aus dem Gehäuse 3 der Pumpe 1 entnommen werden.

Der detaillierte Aufbau des Pumpmoduls ist Figur 5 zu entnehmen.

Ein im Bereich der Deckelwand 19 (bzw. an dieser ausgeformter) befindlicher Ringwulst 52 stellt einen Axial-Anschlag für eine vorzugsweise integral mit der Kolbenstange 16 ausgestaltete Begrenzungsplatte 53 dar. So ist der maximale Hub der Kolben-Zylinder-Einheit 11 durch die Begrenzungsplatte 53 festgelegt und ein Herausfallen des Kolbens 15 aus dem Zylinder 14 formschlüssig vermieden. An der der Bodenwand 17 des Zylinders 14 zugewandten End-Seite der Kolbenstange 16 ist der Kolben 15 vorzugsweise als ein Leichtlaufkolben angeordnet/ausgebildet/fixiert. Der Kolben ist dabei weiter vorzugsweise als eine Kolbenhülse mit bestimmter Hülsenlänge oder als Doppelkolben mit zwei axialbeabstandeten Kolbenplatten (nicht weiter gezeigt) ausgebildet. Dieser weist an seinem distalen und an seinem proximalen Ende (oder distale und proximale Kolbenplatte jeweils) eine als bewegliche Dichtlippe ausgestaltete Dichtung 20 auf Figur 5 gibt zu erkennen, dass jene Dichtungen 20 in einem bevorzugten Ausführungsbeispiel in Umfangsrichtung durchgehend als Ringdichtungen ausgestaltet sind, wodurch eine Unter- und Überdruckbewegung gewährleistet ist. Die Distanz entlang der Längsrichtung L zwischen der distalen und der proximalen Dichtung 20 des Kolbens 15 übersteigt in dem gezeigten Ausführungsbeispiel den Kolbenhub der Kolbenzylindereinheit 11. Somit ist garantiert, dass die Zylinderwandung des Fluidraums / Dosierraums 21 ausschließlich mit der einen, distalen Dichtung 20 in Kontakt ist, wodurch dessen Sterilität sichergestellt ist. Vorzugsweise beträgt der Überschuss zwischen der Distanz entlang der Längsrichtung L zwischen der distalen und der proximalen Dichtung 20 des Kolbens 15 und dem Kolbenhub mindestens 2 mm.

Das Pumpmodul 2 weist zudem eine Griffplatte 54 auf. Diese weist in ihrem Querschnittsprofil eine T-Form auf, wodurch sich eine Art Griffleiste oder Griffbereich 55 ergibt, mittels dem das Pumpmodul 2 leicht greifbar ist und in das Gehäuse 3 einsetzbar oder aus diesem herausnehmbar ist. Im Konkreten ist die Griffleiste 55 am distalen Ende der Kolben-Zylindereinheit 11 so angeordnet, dass sich die Griffleiste 55 quer zur Zylinder-Längsachse L, vorzugsweise senkrecht dazu erstreckt sowie weiter vorzugsweise parallel zu den Schlauchabschnitten 12, 13 verläuft. Die Griffleiste-/platte 54 ist an ihrem auf Seiten des ersten Schlauchabschnitts 12 zugewandten/befindlichen Ende mit einer Hülse oder Öse 56 versehen, die quer zur Griffleiste/-platte vorragt und deren Längsachse parallel zur Griffleiste/-platte verläuft. Die Hülse/Öse 56 ist vorzugsweise integral mit der Griffleiste/-platte 54 ausgestaltet und dient dazu, eine Infusionsleitung (z.B. den Schlauchabschnitt 12) zu fixieren, die durch sie hindurchgeführt wird. So definiert sie einen kreisrunden Hohlabschnitt, in dem die Infusionsleitung nach ihrem Einsetzen anliegt/eingeführt ist. An ihrem auf Seiten des zweiten Schlauchabschnitts 13 zugewandten/befindlichen Ende ist die Griffleiste/-platte 54 mit der Schiebklemme 45 mechanisch gekoppelt. Hierfür sind an der Griffleiste/- platte 54 Rastnocken oder Federzungen mit Rastvorsprüngen 57 angebracht/ausgebildet, wohingegen an der Schiebklemme 45 ein Einsteckschacht angeformt ist. Bei den Rastnocken 57 handelt es sich beispielsweise um quader- oder kugelförmige Vorstehelemente, die in entsprechende Gegenösen/Rücksprünge/Ausnehmungen im Einsteckschacht bzw. dessen Umfangswand der Schiebklemme 45 eingreifen, um diese positionsdefiniert mit der Griffleiste/-platte 54 zu koppeln. Bei jenem Verbinden handelt es sich demnach um einen Formschluss, der über ein Einschnappen der Gegenösen über die Rastnocken 57 realisiert ist.

Die Schiebklemme 45 weist einen starren Teil oder Rahmen 58 und einen beweglichen Teil oder Schieber 59 auf, der im starren Teil oder Rahmen 58 gelagert ist. Der Schieber 59 wie auch der Rahmen 58 sind jeweils mit einem Durchgangsloch/Öffnung ausgeformt, die in einer ersten Schiebeposition deckungsgleich positioniert sind und in einer zweiten Schiebeposition zueinander verschoben sind. Diese klemmen in der zweiten Schiebe-Position/Zustand, in dem die Pumpe 1 nicht fördert bzw. das Pumpmodul 2 nicht in das Gehäuse 3 eingesetzt ist, den ersten Schlauchabschnitt 13 derart ab, dass dessen Luftblasenfreiheit garantiert ist. In der ersten/weiteren Schiebeposition/Zustand wird der bewegliche Teil 59 relativ zu dem starren Teil 58 derart bewegt, dass die eine, erste Öffnung 60, die von dem starren Teil 58 ausgebildet ist, und die andere, zweite Öffnung 61, die von dem beweglichen Teil 59 der Schiebklemme 45 ausgebildet ist, bündig zueinander sind (sich überdecken) und so einen Fluidfluss durch den zweiten Schlauchabschnitt 13 freigeben, der durch die beiden Durchgangslöcher/Öffnungen hindurchgeführt ist.

Der erfindungsgemäße Vorteil, dass die Infusionspumpe 1 für sämtliche Anwendungen einer Infusion, das heißt solche mit hohem Fördervolumen und solche mit hohen Genauigkeitsanforderungen, einsetzbar ist, wird weiter dadurch verstärkt, dass sämtliche Infusionssets an die Pumpe anschließbar sind. Beispielhaft hierfür offenbart Figur 6 die Kopplung des ersten Schlauchabschnitts 12 mit einer Tropfkammer 50. So ist es möglich, das Pumpmodul 2 mit einer Tropfkammer 50 zu koppeln und diese Einheit dann in das Gehäuse 3 einzusetzen. Die Tropfkammer 50, die im Regelfall Bestandteil jedes Infusionssystems ist, ist somit direkt stromabwärts zum Pumpmodul 2 anordenbar, wodurch die Kompaktheit des gesamten Infusionssystems wahlweise erhöht ist. Sie dient der regulierten Tropfenbildung der verabreichten Flüssigkeit sowie der Vermeidung einer Luftbläschenbildung.

In Figur 7 ist eine weitere Ausführungsform mit einem anderen Infusionsset dargestellt. So weist die Infusionsleitung hier stromabwärts zum Pumpmodul 2 einen Spike 62 auf. Dieser stellt das Verbindungsstück dar, das eine zuverlässige Verbindung zwischen einem externen Infusionsbehälter und den Infusionsleitungen herstellt. Der Spike 62 ist in einen Einstechdorn 63 und einen Haltesitz 64 einteilbar. Zwischen diesen ist ein Spikeanschlag 65 ausgebildet, der ein sicheres Sitzen des Spikes 62 ermöglicht.

In Figur 8 ist der erste Schlauchabschnitt 12, wie in Figur 3, mit dem Einlass-Luer-Lock-Anschluss / Luer-Lock-Innenkegel 24 versehen. Die Schiebeklemme 45 ist hierbei in einem solchen Zustand, dass sie ein Durchfließen des zweiten Schlauchabschnitts 13 zulässt. In diesem Zustand, in dem ein Durchfließen ermöglicht ist, ist das Pumpmodul 2 in das Gehäuse 3 eingesetzt, wie in Figur 2 und in Figur 9 dargestellt. Figur 9 zeigt hierbei die Ausführungsform, in der der Spike 62 mit dem ersten Schlauchabschnitt 12 verbunden ist.

### Bezugszeichenliste

- 1: Infusionspumpe
- 2: Pumpmodul
- 3: Gehäuse
- 4: Gehäuseunterteil
- 5: Gehäusedeckel
- 6: Verschlussklappe
- 7: Gehäuseboden
- 8: Anzeigeelemente
- 9: Bedienungselemente
- 10: Anzeigedisplay
- 11: Kolben-Zylinder-Einheit
- 12: erster Schlauchabschnitt
- 13: zweiter Schlauchabschnitt
- 14: Zylinder
- 15: Kolben
- 16: Kolbenstange
- 17: Bodenwand
- 18: Umfangswand
- 19: Deckelwand
- 20: Dichtung
- 21: Fluidraum / Dosierraum
- 22: Einlaufbohrung
- 23: Auslaufbohrung
- 24: Einlass-Luer-Lock-Anschluss, Luer-Lock-Innenkegel
- 25: Auslass-Luer-Lock-Anschluss, Luer-Lock-Außenkegel
- 26: Antrieb, Linearmotor
- 27: Zwischenwand
- 28: Steuereinheit
- 29: Zwischenwand
- 30: Antrieb, Linearmotor
- 31: Kipphebel
- 32: Druckstempel, Ventileinrichtung
- 33: Druckstempel, Ventileinrichtung
- 34: Mitnehmer
- 35: Kipplager
- 36: Schwenklager
- 37: Schwenklager
- 38: Querarm
- 39: Druckfeder
- 40: Druckfeder
- 41: Verschlussmechanismus
- 42: Riegelstift
- 43: Positionierungszapfen
- 44: Luftsensor
- 45: Schiebeklemme
- 46: Kuppelelement
- 47: Gegenelement
- 48: Akku
- 49: USB-Schnittstelle
- 50: Tropfkammer
- 51: Spike
- 52: Ringwulst
- 53: Begrenzungsplatte
- 54: Griffplatte
- 55: Griffbereich
- 56: Hülse
- 57: Rastnocken
- 58: starrer Teil der Schiebeklemme
- 59: beweglicher Teil der Schiebeklemme
- 60: erste Öffnung der Schiebeklemme
- 61: zweite Öffnung der Schiebeklemme
- 62: Spike
- 63: Einstechdorn
- 64: Haltesitz
- 65: Spikeanschlag
- 66: Übersetzungsgetriebe

- L: Längsrichtung, Achsrichtung

## Patentansprüche

1. Infusionspumpe (1) mit einem Pumpmodul (2) umfassend eine Kolben-Zylinder-Einheit (11), die dafür angepasst ist, mittels eines Antriebsmechanismus (26, 66) der Infusionspumpe (1) bestehend aus einem elektrischen Antriebsmotor (26) sowie einem Übersetzungsgetriebe (66) zur Transformation der Rotationsbewegung des Antriebsmotors (26) in eine Translationsbewegung/Hin-/HerBewegung des im Zylinder (14) verschiebbar gelagerten Kolbens (15) oder bestehend aus einem Linearantrieb/-motor (26) betätigt zu werden, der weiter über eine Steuer-/Regeleinheit (28) der Infusionspumpe (1) gesteuert/geregelt ist, sowie mit einer Ventileinrichtung (32, 33), welche dafür angepasst ist, um im Fall eines Pumpförderhubs eine Verbindung (12) zwischen der Kolben-Zylinder-Einheit (11) als Flüssigkeit-Zwischenspeicher (11) und einem Versorgungsanschluss (24, 50, 62) zu einem separaten Flüssigkeitsgroßvolumen zu unterbrechen sowie eine Verbindung (13) zwischen der Kolben-Zylinder-Einheit (11) und einem Patientenanschluss (25) freizugeben und im Fall eines Pumpsaughubs die Verbindung (12) zwischen der Kolben-Zylinder-Einheit (11) und dem Versorgungsanschluss (24, 50, 62) zu dem separaten Flüssigkeitsgroßvolumen freizugeben sowie die Verbindung (13) zwischen der Kolben-Zylinder-Einheit (11) und dem Patientenanschluss (25) zu sperren, wobei die Steuereinheit (28) eine motorisch angetriebene Kipphebeleinheit (31 - 40) als Ventileinrichtung aktiviert, die in einer ersten Kippstellung den ersten Schlauchabschnitt (12) zusammenquetscht und den zweiten Schlauchabschnitt (13) freigibt und in einer zweiten Kippstellung den zweiten Schlauchabschnitt (13) zusammenquetscht und den ersten Schlauchabschnitt (12) freigibt.

2. Infusionspumpe (1) nach Anspruch 1, **dadurch gekennzeichnet, dass** die Ventileinrichtung (32, 33) an die Steuer-/Regeleinheit (28) angeschlossen ist und dafür angepasst ist, um in geregelter/gesteuerter Weise im Fall eines Pumpförderhubs eine Verbindung (12) zwischen der Kolben-Zylinder-Einheit (11) als Flüssigkeit-Zwischenspeicher und des Versorgungsanschlusses (24, 50, 62) zu einem separaten Flüssigkeitsgroßvolumen zu unterbrechen sowie eine Verbindung (13) zwischen der Kolben-Zylinder-Einheit (11) und einem Patientenanschluss (25) freizugeben und im Fall eines Pumpsaughubs die Verbindung (12) zwischen der Kolben-Zylinder-Einheit (11) und dem Versorgungsanschluss (24, 50, 62) zu dem separaten Flüssigkeitsgroßvolumen freizugeben sowie die Verbindung (13) zwischen der Kolben-Zylinder-Einheit (11) und dem Patientenanschluss (25) zu sperren oder die Ventileinrichtung passiv betätigbare Ventilelemente bevorzugt in Form zweier Rückschlagventile hat, welche in vorzugsweise separaten, als Einweg-Artikel ausgebildeten Verbindungsleitungen (12, 13) zum Patientenanschluss (25) und zum Flüssigkeitsgroßvolumenanschluss (24) untergebracht sind, die in die Infusionspumpe (1) einlegbar sind.

3. Infusionspumpe (1) nach Anspruch 1, **dadurch gekennzeichnet, dass** die Ventileinrichtung (32, 33) mit aktiv betätigbaren Ventilelementen, vorzugsweise zwei Schlauchquetschen/Pressen (32, 33) versehen ist, die auf flexibel deformierbare Abschnitte der beiden Verbindungsleitungen (12, 13) für deren dichtendes Abdrücken einwirken und die bevorzugt in der Infusionspumpe (1) als deren fester Bestandteil untergebracht sind.

4. Infusionspumpe (1) nach einem der vorstehenden Ansprüche, **dadurch gekennzeichnet, dass** eine Kolbenstange (16) der Kolben-Zylinder-Einheit (11) ein Kuppelelement (46) zum lösbaren Kuppeln mit einem entsprechenden Gegenelement (47) des Antriebsmechanismus (26) der Infusionspumpe (1) aufweist.

5. Infusionspumpe (1) nach einem der vorstehenden Ansprüche, **dadurch gekennzeichnet, dass** der erste Schlauchabschnitt (12) und/oder der zweite Schlauchabschnitt (13) am jeweils der Kolben-Zylinder-Einheit (11) gegenüber liegenden Ende mit einem Luer-Lock-Kupplungsstück (24, 25) oder einer Tropfkammer (50) oder einem Spike (62) versehen ist/sind.

6. Infusionspumpe (1) nach einem der vorstehenden Ansprüche, **dadurch gekennzeichnet, dass** die Steuereinheit (28) und der Antriebsmechanismus (26) an einem Gehäuseteil (4) der Infusionspumpe (1) angeordnet sind und das Pumpmodul (2) anwenderseitig austauschbar an/in dem Gehäuseteil (4) anzuordnen ist.

7. Infusionspumpe (1) nach einem der vorstehenden Ansprüche, **dadurch gekennzeichnet, dass** die Steuer-/Regeleinheit (28) derart mit dem Antriebsmechanismus (26) für die Kolben-Zylinder-Einheit (11) gekoppelt ist, dass sie ein im Wesentlichen fluiddichtes Zusammenquetschen des ersten Schlauchabschnitts (12) bewirkt, wenn Fluid aus der Kolben-Zylinder-Einheit (11) ausgetrieben wird, und sie ein im Wesentlichen fluiddichtes Zusammenquetschen des zweiten Schlauchabschnitts (13) bewirkt, wenn Fluid in die Kolben-Zylinder-Einheit (11) eingesaugt wird.

8. Infusionspumpe (1) nach einem der Ansprüche 4 bis 7, **dadurch gekennzeichnet, dass** der Linearantrieb/-motor (26) in der Bewegungsachse eines Kolbens (15) und/oder der Kolbenstange (16) angeordnet ist.

9. Infusionspumpe (1) nach einem der vorstehenden Ansprüche, **dadurch gekennzeichnet, dass** diese eine Aufnahme für das Pumpmodul (2) aufweist, die insbesondere mittels eines Verschlusses (6) zu verschließen ist.

10. Infusionspumpe (1) nach Anspruch 9, **dadurch gekennzeichnet, dass** der Verschluss (6) in einer die Aufnahme verschließenden Stellung über eine Verriegelungseinheit (41) mit dem Gehäuseteil (3, 4, 5) verriegelbar ist.

11. Infusionspumpe (1) nach Anspruch 1, **dadurch gekennzeichnet, dass** schwenkbar an der Kipphebeleinheit (31 - 40) angeordnete Druckstempel (32, 33) vorgesehen sind, insbesondere ein mit dem ersten Schlauchabschnitt (12) zusammenwirkender Einlassstempel (32) und ein mit dem zweiten Schlauchabschnitt (13) zusammenwirkender Auslassstempel (33), wobei zumindest ein Druckstempel (32, 33) mittels einer Vorspanneinheit (39, 40), insbesondere mittels einer Druckfeder (39, 40), in eine den jeweiligen Schlauchabschnitt (12, 13) freigebende, insbesondere nicht kontaktierende, Position vorgespannt ist.

12. Infusionspumpe (1) nach einem der vorstehenden Ansprüche, **dadurch gekennzeichnet, dass** diese eine Energiespeichereinheit (48), insbesondere einen Akku (48), hat.

13. Infusionspumpe (1) nach einem der vorstehenden Ansprüche, **dadurch gekennzeichnet, dass** das Pumpmodul (2) eine Schiebeklemme (45) aufweist, die derart einstellbar ist, dass sie einen Fluidfluss in dem ersten und/oder dem zweiten Schlauchabschnitt (12, 13) wahlweise freigibt oder sperrt.

14. Infusionspumpe (1) nach Anspruch 13, **dadurch gekennzeichnet, dass** an einem der Schiebeklemme (45) abgewandten Ende des Pumpmoduls (2) eine Hülse (56) angeordnet ist, die dazu vorbereitet ist, den ersten und/oder den zweiten Schlauchabschnitt (12, 13) zu fixieren.

## Claims

1. An infusion pump (1) with a pump module (2) comprising a piston-cylinder unit (11) which is adapted to be actuated via a drive mechanism (26, 66) of the infusion pump (1) consisting of an electric drive motor (26) and a transmission gearing (66) for transforming the rotational movement of the drive motor (26) into a translational movement/ back/ forth movement of the piston (15) displaceably mounted in the cylinder (14), or consisting of a linear drive/motor (26), which is further controlled/regulated via a control/regulating unit (28) of the infusion pump (1), as well as with a valve device (32, 33) which is adapted to interrupt a connection (12) between the piston-cylinder unit (11) as temporary fluid storage (11) and a feed terminal (24, 50, 62) to a separate large liquid volume and to release a connection (13) between the piston-cylinder unit (11) and a patient terminal (25) and, in the case of a pump suction stroke, to release the connection (12) between the piston-cylinder unit (11) and the feed terminal (24, 50, 62) to the separate large liquid volume and to block the connection (13) between the piston-cylinder unit (11) and the patient terminal (25), wherein the control unit (28) activates a motor-driven tilting lever unit (31 - 40) as a valve device, which in a first tilt position squeezes the first hose portion (12) together and releases the second hose portion (13) and in a second tilt position squeezes the second hose portion (13) together and releases the first hose portion (12).

2. The infusion pump (1) according to claim 1, **characterized in that** the valve device (32, 33) is connected to the control/regulating unit (28) and is adapted to interrupt in a regulated/controlled manner, in the case of a pump delivery stroke, a connection (12) between the piston-cylinder unit (11) as temporary fluid storage and the feed terminal (24, 50, 62) to a separate large liquid volume as well as to release a connection (13) between the piston-cylinder unit (11) and a patient terminal (25) and, in the case of a pump suction stroke, to release the connection (12) between the piston-cylinder unit (11) and the feed terminal (24, 50, 62) to the separate large liquid volume as well as to block the connection (13) between the piston-cylinder unit (11) and the patient terminal (25), or the valve device has passively actuable valve elements, preferably in the form of two non-return valves, which are accommodated in preferably separate connection lines (12, 13), designed as disposable articles, to the patient terminal (25) and to the large liquid volume connection (24), which are insertable into the infusion pump (1).

3. The infusion pump (1) according to claim 1, **characterized in that** the valve device (32, 33) is provided with actively operable valve elements, preferably two hose squeezers/presses (32, 33), which act on flexibly deformable portions of the two connection lines (12, 13) for being squeezed in a sealing manner and which are preferably accommodated in the infusion pump (1) as a fixed component thereof.

4. The infusion pump (1) according to one of the preceding claims, **characterized in that** a piston rod (16) of the piston-cylinder unit (11) has a coupling element (46) for detachable coupling to a corresponding counter element (47) of the drive mechanism (26) of the infusion pump (1).

5. The infusion pump (1) according to one of the preceding claims, **characterized in that** the first hose portion (12) and/or the second hose portion (13) is/are each provided with a Luer lock coupling piece (24, 25) or a drip chamber (50) or a spike (62) at the end opposite the piston-cylinder unit (11).

6. The infusion pump (1) according to one of the preceding claims, **characterized in that** the control unit (28) and the drive mechanism (26) are arranged on a housing part (4) of the infusion pump (1) and the pump module (2) is to be arranged on/in the housing part (4) to be exchangeable by the user.

7. The infusion pump (1) according to one of the preceding claims, **characterized in that** the control/regulating unit (28) is coupled to the drive mechanism (26) for the piston-cylinder unit (11) in such a way that it effects a substantially fluid-tight squeezing of the first hose portion (12) when fluid is expelled from the piston-cylinder unit (11), and it effects a substantially fluid-tight squeezing of the second hose portion (13) when fluid is sucked into the piston-cylinder unit (11).

8. The infusion pump (1) according to one of claims 4 to 7, **characterized in that** the linear drive/motor (26) is arranged in the axis of movement of a piston (15) and/or of the piston rod (16).

9. The infusion pump (1) according to one of the preceding claims, **characterized in that** it has a receptacle for the pump module (2), which is to be closed in particular via a closure (6).

10. The infusion pump (1) according to claim 9, **characterized in that** the closure (6) can be locked to the housing part (3, 4, 5) in a position closing the receptacle via a locking unit (41).

11. The infusion pump (1) according to claim 1, **characterized in that** pressure plungers (32, 33) arranged pivotably on the tilting lever unit (31 - 40) are provided, in particular an inlet plunger (32) cooperating with the first hose portion (12) and an outlet plunger (33) cooperating with the second hose portion (13), wherein at least one pressure plunger (32, 33) is prestressed via a prestressing unit (39, 40), in particular via a compression spring (39, 40), into a position releasing the respective hose portion (12, 13), in particular a non-contacting position.

12. The infusion pump (1) according to one of the preceding claims, **characterized in that** it has an energy storage unit (48), in particular a rechargeable battery (48).

13. The infusion pump (1) according to one of the preceding claims, **characterized in that** the pump module (2) has a sliding clamp (45) which is adjustable in such a way that it selectively releases or blocks a fluid flow in the first and/or the second hose portion (12, 13).

14. The infusion pump (1) according to claim 13, **characterized in that** a sleeve (56) is arranged at an end of the pump module (2) facing away from the sliding clamp (45), wherein the sleeve is prepared for fixating the first and/or the second hose portion (12, 13).

## Revendications

1. Pompe à perfusion (1) avec un module de pompe (2) comprenant un ensemble piston-cylindre (11) qui est adaptée afin d'être actionnée au moyen d'un mécanisme d'entraînement (26, 66) de la pompe à perfusion (1) consistant en un moteur d'entraînement (26) électrique ainsi qu'en un engrenage de transmission (66) pour la transformation du mouvement de rotation du moteur d'entraînement (26) en un mouvement de translation/mouvement de va-et-vient du piston (15) logé de manière coulissante dans le cylindre (14) ou consistant en un moteur/entraînement linéaire (26) qui est commandé/régulé en outre par le biais d'un dispositif de commande/régulation (28) de la pompe à perfusion (1), ainsi qu'avec un dispositif de soupape (32, 33) qui est adapté afin d'interrompre, dans le cas d'une course de refoulement de pompe, une liaison (12) entre l'ensemble piston-cylindre (11) servant d'accumulateur intermédiaire de liquide (11) et un raccord d'alimentation (24, 50, 62) vers un grand volume de liquide séparé ainsi que de libérer une liaison (13) entre l'ensemble piston-cylindre (11) et un raccord patient (25) et de libérer, dans le cas d'une course d'aspiration de pompe, la liaison (12) entre l'ensemble piston-cylindre (11) et le raccord d'alimentation (24, 50, 62) vers le grand volume de liquide séparé ainsi que de bloquer la liaison (13) entre l'ensemble piston-cylindre (11) et le raccord patient (25), dans lequel le dispositif de commande (28) active une unité de levier de basculement (31-40) entraînée de manière motorisée comme dispositif de soupape qui écrase la première section de tuyau (12) et libère la seconde section de tuyau (13) dans une première position de basculement et écrase la seconde section de tuyau (13) et libère la première section de tuyau (12) dans une seconde position de basculement.

2. Pompe à perfusion (1) selon la revendication 1, **caractérisée en ce que** le dispositif de soupape (32, 33) est raccordé au dispositif de commande/régulation (28) et est adapté afin d'interrompre de manière régulée/commandée, dans le cas d'une course de refoulement de pompe, une liaison (12) entre l'ensemble piston-cylindre (11) servant d'accumulateur intermédiaire de liquide et le raccord d'alimentation (24, 50, 62) vers un grand volume de liquide séparé ainsi que de libérer une liaison (13) entre l'ensemble piston-cylindre (11) et un raccord de patient (25) et de libérer, dans le cas d'une course d'aspiration de pompe, la liaison (12) entre l'ensemble piston-cylindre (11) et le raccord d'alimentation (24, 50, 62) vers le grand volume de liquide séparé ainsi que de bloquer la liaison (13) entre l'ensemble piston-cylindre (11) et le raccord patient (25) ou le dispositif de soupape présente des éléments de soupape actionnables de manière passive de préférence sous la forme de deux clapets antiretour qui sont logés dans des conduites de liaison (12, 13) séparées de préférence, réalisées comme articles à usage unique au raccord patient (25) et au raccord de grand volume de liquide (24) qui peuvent être introduites dans la pompe à perfusion (1).

3. Pompe à perfusion (1) selon la revendication 1, **caractérisée en ce que** le dispositif de soupape (32, 33) est pourvu d'éléments de soupape actionnables activement, de préférence deux écrasements/pressages (32, 33) qui agissent sur des sections déformables de manière flexible des deux conduites de liaison (12, 13) pour leur épreuve à la pression étanche et qui sont logés de préférence dans la pompe à perfusion (1) comme leur constituant fixe.

4. Pompe à perfusion (1) selon l'une quelconque des revendications précédentes, **caractérisée en ce qu'**une tige à piston de l'ensemble piston-cylindre (11) présente un élément de couplage (46) pour le couplage amovible avec un contre-élément (47) correspondant du mécanisme d'entraînement (26) de la pompe à perfusion (1).

5. Pompe à perfusion (1) selon l'une quelconque des revendications précédentes, **caractérisée en ce que** la première section de tuyau (12) et/ou la seconde section de tuyau (13) est/sont pourvues au niveau de l'extrémité se trouvant respectivement en face de l'ensemble piston-cylindre (11) d'une pièce de couplage Luer Lock (24, 25) ou d'une chambre de gouttes (50) ou d'une pointe (62).

6. Pompe à perfusion (1) selon l'une quelconque des revendications précédentes, **caractérisée en ce que** le dispositif de commande (28) et le mécanisme d'entraînement (26) sont agencés au niveau d'une partie de boîtier (4) de la pompe à perfusion (1) et le module de pompe (2) doit être agencé de manière interchangeable côté utilisateur au niveau/dans la partie de boîtier (4).

7. Pompe à perfusion (1) selon l'une quelconque des revendications précédentes, **caractérisée en ce que** le dispositif de commande/régulation (28) est couplé au mécanisme d'entraînement (26) pour l'ensemble piston-cylindre (11) de telle manière qu'il entraîne un écrasement sensiblement étanche au fluide de la première section de tuyau (12) lorsque du fluide est expulsé de l'ensemble piston-cylindre (11), et il entraîne un écrasement sensiblement étanche au fluide de la seconde section de tuyau (13) lorsque du fluide est aspiré dans l'ensemble piston-cylindre (11).

8. Pompe à perfusion (1) selon l'une quelconque des revendications 4 à 7, **caractérisée en ce que** le moteur/l'entraînement linéaire (26) est agencé dans l'axe de déplacement d'un piston (15) et/ou de la tige de piston (16).

9. Pompe à perfusion (1) selon l'une quelconque des revendications précédentes, **caractérisée en ce que** celle-ci présente un logement pour le module de pompe (2) qui doit être fermé en particulier au moyen d'une fermeture (6).

10. Pompe à perfusion (1) selon la revendication 9, **caractérisée en ce que** la fermeture (6) est verrouillable avec la partie de boîtier (3, 4, 5) par le biais d'une unité de verrouillage (41) dans une position fermant le logement.

11. Pompe à perfusion (1) selon la revendication 1, **caractérisée en ce que** des pistons de pression (32, 33) agencés de manière pivotante au niveau de l'unité de levier de basculement (31-40) sont prévus, en particulier un piston d'entrée (32) interagissant avec la première section de tuyau (12) et un piston de sortie (33) interagissant avec la seconde section de tuyau (13), dans laquelle au moins un piston de pression (32, 33) est précontraint au moyen d'une unité de précontrainte (39, 40), en particulier au moyen d'un ressort de pression (39, 40) dans une position libérant la section de tuyau (12, 13) respective, en particulier sans la toucher.

12. Pompe à perfusion (1) selon l'une quelconque des revendications précédentes, **caractérisée en ce que** celle-ci présente une unité d'accumulateur d'énergie (48), en particulier une batterie.

13. Pompe à perfusion (1) selon l'une quelconque des revendications précédentes, **caractérisée en ce que** le module de pompe (2) présente une pince coulissante (45) qui est réglable de telle manière qu'elle libère ou bloque au choix un flux de fluide dans la première et/ou la seconde section de tuyau (12, 13).

14. Pompe à perfusion (1) selon la revendication 13, **caractérisée en ce qu'**une douille (56) est agencée au niveau d'une extrémité opposée de la pince coulissante (45) du module de pompe (2), douille qui est préparée afin de fixer la première et/ou la seconde section de tuyau (12, 13).
